# EUROPEAN PATENT SPECIFICATION

(11) **EP 4 033 989 B1**
(45) Date of publication and mention of the grant of the patent: **17.04.2024**
(21) Application number: 20768604.9
(22) Date of filing: 10.09.2020
(51) Int. Cl.: A61B 8/12, B06B 1/06, A61B 8/00

(54) **INTRALUMINAL IMAGING DEVICE**
INTRALUMINALE BILDGEBUNGSVORRICHTUNG
DISPOSITIF D'IMAGERIE INTRALUMINALE

(30) Priority: 26.09.2019 US 201962906220 P
(43) Date of publication of application: 03.08.2022
(73) Proprietor: Koninklijke Philips N.V., 5656 AG Eindhoven (NL)
(72) Inventor: ROBINSON, Andrew, Lee, 5656 AE Eindhoven (NL); HALILI, Reynaldo, Borja, 5656 AE Eindhoven (NL)
(74) Representative: Philips Intellectual Property & Standards
(86) International application number: PCT/EP2020/075286
(87) International publication number: WO 2021/058286

(56) References cited:
- WO-A1-2018/178382
- US-A1- 2013 286 786

## Description

### TECHNICAL FIELD

The present disclosure relates generally to intraluminal imaging devices and, in particular, to intraluminal imaging devices comprising multiple segments of conductors of different sizes positioned within corresponding spaces of one or more flexible elongate members.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. An IVUS device including one or more ultrasound transducers is passed into the vessel and guided to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

Phased array (also known as synthetic-aperture) IVUS catheters are one of the two types of IVUS devices commonly used today, the other type being the rotational IVUS catheter. Phased array IVUS catheters carry a scanner assembly that includes an array of ultrasound transducers positioned and distributed around its perimeter or circumference along with one or more integrated circuit controller chips mounted adjacent to the transducer array. The controllers select individual acoustic elements (or groups of elements) for transmitting a pulse of acoustic energy and for receiving the ultrasound echo signal corresponding to the transmitted ultrasound energy. By stepping through a sequence of transmit-receive pairs, the phased array IVUS system can synthesize the effect of a mechanically scanned ultrasound transducer but without moving parts (hence the solid-state designation). Since there is no rotating mechanical element, the transducer array can be placed in direct contact with the blood and vessel tissue with minimal risk of vessel trauma and without a need for an additional housing between the rotating element and the vessel lumen.

IVUS catheters typically include one or more conductors positioned within a flexible elongate member configured to be inserted into the vasculature of the patient. The one or more conductors carry electrical signals between a processor circuit of, for example, a patient interface module (PIM), and the scanner assembly positioned at the distal end of the flexible elongate member. IVUS catheters also typically include other components within the flexible elongate member, including structural supports and/or inner members configured to define different lumens or spaces within the flexible elongate member. Because the flexible elongate member must be small enough to fit within the vasculature, and because the other components within the flexible elongate member limit the space within the flexible elongate member, the space available for the conductors may be limited. In that regard, the space available for the conductors may be particularly limited at certain positions along the length of the flexible elongate member. Accordingly, conventional IVUS catheters include conductors (e.g., wires) that are small enough to be positioned within even the most limited spaces of the flexible elongate member. However, these small conductors exhibit high signal loss, which limits the speed and/or quality of information transmitted and/or received by the IVUS imaging system.

WO 2018/178382 A1 discloses an intraluminal imaging device that includes a flexible elongate member for insertion into a body lumen within a patient. The imaging device has an imaging assembly that is disposed at a distal portion of the flexible elongate member. The imaging assembly has a plurality of ultrasound transducer elements disposed around the longitudinal axis. The imaging assembly further includes an annularly shaped integrated circuit controller in communication with the plurality of ultrasound transducer elements.

US 2013/0286786 A1 discloses an ultrasound transducer array that includes a substrate, a plurality of groove-like recesses arranged at a predetermined interval on one surface of the substrate, a cell region arranged between the recesses on the one surface side of the substrate, a flexible film configured to cover the substrate and the cell region and having fragility lower than fragility of the substrate, and a dividing groove having a width smaller than a width of the recess and reaching from the other surface of the substrate to the flexible film in the recess.

### SUMMARY

Aspects of the present disclosure provide intraluminal imaging devices that include multiple conductor segments having conductors of different sizes at particular locations along the length of the intraluminal imaging device. For example, the intraluminal imaging device may include a scanner assembly at a distal end of the imaging device, and a connector at a proximal end of the imaging device. The imaging transducer and the connector are electrically coupled by two or more conductor segments, each comprising a plurality of conductors. The intraluminal imaging device may include a plurality of lengths or sections, including a first section comprising a flexible cable with a first space of a first size, and a second section comprising a flexible elongate member defining a second space of a smaller second size. The cable includes a first plurality of conductors of a first cross-sectional size forming a first conductor segment. The first plurality of conductors is positioned within the larger first space. A second plurality of conductors of a smaller second cross-sectional size forming a second conductor segment is positioned within the smaller second space. By including the first conductor segment having the larger conductor size, the signal loss can be reduced, which may increase the speed, reliability, and/or quality of the information and electrical signals transmitted between the connector and the imaging transducer.

In one embodiment, an intraluminal ultrasound imaging device according to claim 1 is provided.

In some embodiments, the first plurality of conductors comprises a first twisted group of conductors and a first power line, and the second plurality of conductors comprises a second twisted group of conductors electrically coupled to the first twisted group of conductors, and a second power line electrically coupled to the first power line. In some embodiments, the second plurality of conductors comprises eight conductors forming a first twisted quad and a second twisted quad. In some embodiments, the second plurality of conductors comprises at least one twisted group of wires, and at least one non-twisted wire. In some embodiments, the IVUS imaging device further includes a hub coupled to the distal portion of the cable and the proximal portion of the flexible elongate member. In one aspect, the first plurality of conductors is electrically coupled to the second plurality of conductors within the hub. In another aspect, the hub comprises a housing and a printed circuit board positioned within the housing. In one aspect, the printed circuit board comprises a first set of contacts and a second set of contacts. In another aspect, the first plurality of conductors are coupled to the first set of contacts and the second plurality of conductors are coupled to the second set of contacts.

In some embodiments, the first plurality of conductors and the second plurality of conductors are impedance balanced. In some embodiments, the IVUS imaging device further includes a core wire positioned within the flexible elongate member and defining a space between an outer surface of the core wire and an inner surface of the flexible elongate member. In one aspect, the second plurality of conductors is positioned within the space. In some embodiments, the IVUS imaging device further includes: a guidewire exit port disposed in the flexible elongate member proximal to the ultrasound transducer array; and a third plurality of conductors extending distally from the guidewire exit port within the distal portion of the flexible elongate member. In one aspect, a conductor of the third plurality of conductors with a third cross section is electrically coupled to the conductor of the second plurality of conductors with the second cross section. In another aspect, the third cross section is larger than the second cross section. In some embodiments, the IVUS imaging device further includes a flexible inner member positioned within the space and adjacent to the core wire, the flexible inner member extending distally from the guidewire exit port.

According to another embodiment of the present disclosure, an intravascular ultrasound (IVUS) imaging system according to claim 11 is provided.

In some embodiments, the processor circuit is configured to generate an ultrasound image based on the processed electrical signals. In some embodiments, the processor circuit is communicatively coupled to display, and wherein the processor circuit is configured to output an intraluminal cross-sectional image based on the electrical signals to a display. In some embodiments, the processor circuit is configured to perform an analog-to-digital conversion on the electrical signals.

In some embodiments, the IVUS imaging system further includes: a patient interface module (PIM) configured to be coupled to the electrical connector of the intraluminal ultrasound imaging device to receive the electrical signals; and a console communicatively coupled to the PIM and configured to receive the electrical signals from the PIM. In some embodiments, the processor circuit is disposed within the console. In some embodiments, the processor circuit is disposed within the PIM.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1A is a diagrammatic schematic view of an intraluminal imaging system, according to aspects of the present disclosure.
Fig. 1B is a schematic diagram of a processor circuit, according to embodiments of the present disclosure.
Fig. 2 is a diagrammatic view of the top of a scanner assembly in a flat configuration, according to aspects of the present disclosure.
Fig. 3 is a diagrammatic perspective view of the scanner assembly shown in Fig. 2 in a rolled configuration around a support member, according to aspects of the present disclosure.
Fig. 4 is a diagrammatic cross-sectional side view of a scanner assembly in a rolled configuration around a support member, according to aspects of the present disclosure.
Fig. 5 is a top plan view of an intravascular ultrasound (IVUS) imaging catheter, according to aspects of the present disclosure.
Fig. 6 is a diagrammatic cross-sectional view of the IVUS imaging catheter shown in Fig. 5, taken along line 6-6, according to aspects of the present disclosure.
Fig. 7 is a diagrammatic cross-sectional view of the IVUS imaging catheter shown in Fig. 5, taken along line 7-7, according to aspects of the present disclosure.
Fig. 8 is a magnified, top plan view of the IVUS imaging catheter shown in Fig. 5, according to aspects of the present disclosure.
Fig. 9 is a diagrammatic cross-sectional view of the IVUS imaging catheter shown in Figs. 5 and 8, taken along line 9-9, according to aspects of the present disclosure.
Fig. 10 is a diagrammatic cross-sectional view of the IVUS imaging catheter shown in Figs. 5 and 8, taken along line 10-10, according to aspects of the present disclosure.
Fig. 11 is a diagrammatic cross-sectional view of the IVUS imaging catheter shown in Figs. 5 and 8, taken along line 11-11, according to aspects of the present disclosure.
Fig. 12 is a diagrammatic cross-sectional view of the IVUS imaging catheter shown in Figs. 5 and 8, taken along line 12-12, according to aspects of the present disclosure.
Fig. 13 is a diagrammatic cross-sectional view of the IVUS imaging catheter shown in Figs. 5 and 8, taken along line 13-13, according to aspects of the present disclosure.
Fig. 14 is a diagrammatic view of a first plurality of conductors electrically coupled to a second plurality of conductors within a hub of an IVUS imaging catheter, according to aspects of the present disclosure.
Fig. 15 is a diagrammatic view of a first plurality of conductors electrically coupled to a second plurality of conductors within a hub of an IVUS imaging catheter, wherein the hub includes a circuit board, according to aspects of the present disclosure.
Fig. 16 is a diagrammatic cross-sectional view of a conductor bundle having conductors of different cross-sectional sizes, according to an embodiment of the present disclosure.
Fig. 17 is a flow diagram of a method for assembling a multi-segment intraluminal imaging device, according to aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

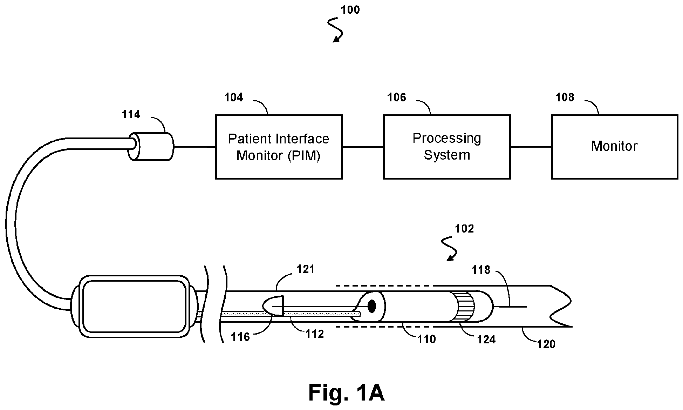

Fig. 1A is a diagrammatic schematic view of an ultrasound imaging system 100, according to aspects of the present disclosure. The ultrasound imaging system 100 can be an intraluminal imaging system. In some instances, the system 100 can be an intravascular ultrasound (IVUS) imaging system. The system 100 may include an intraluminal imaging device 102 such as a catheter, guide wire, or guide catheter, a patient interface module (PIM) 104, a processing system or console 106, and a monitor 108. The intraluminal imaging device 102 can be an ultrasound imaging device. In some instances, the device 102 can be IVUS imaging device, such as a solid-state IVUS device.

At a high level, the IVUS device 102 emits ultrasonic energy, or ultrasound signals, from a transducer array 124 included in scanner assembly 110 mounted near a distal end of the catheter device. The ultrasonic energy is reflected by tissue structures in the medium, such as a vessel 120, or another body lumen surrounding the scanner assembly 110, and the ultrasound echo signals are received by the transducer array 124. In that regard, the device 102 can be sized, shaped, or otherwise configured to be positioned within the body lumen of a patient. The PIM 104 transfers the received echo signals to the console or computer 106 where the ultrasound image (including the flow information) is reconstructed and displayed on the monitor 108. The processing system 106 can include a processor and a memory. The processing system 106 can be operable to facilitate the features of the IVUS imaging system 100 described herein. For example, the processing system 106 can execute computer readable instructions stored on the non-transitory tangible computer readable medium.

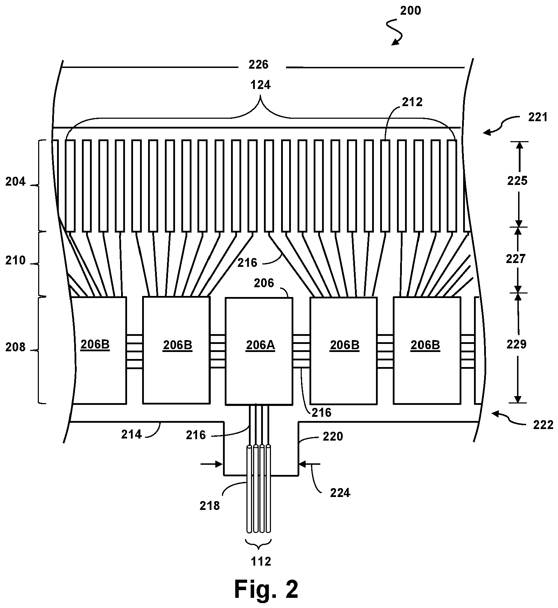

The PIM 104 facilitates communication of signals between the processing system 106 and the scanner assembly 110 included in the IVUS device 102. This communication includes the steps of: (1) providing commands to integrated circuit controller chip(s) 206A, 206B, illustrated in Fig. 2, included in the scanner assembly 110 to select the particular transducer array element(s), or acoustic element(s), to be used for transmit and receive, (2) providing the transmit trigger signals to the integrated circuit controller chip(s) 206A, 206B included in the scanner assembly 110 to activate the transmitter circuitry to generate an electrical pulse to excite the selected transducer array element(s) 212, and/or (3) accepting amplified echo signals received from the selected transducer array element(s) 212 via amplifiers included on the integrated circuit controller chip(s) 206 of the scanner assembly 110. In some embodiments, the PIM 104 performs preliminary processing of the echo data prior to relaying the data to the console 106. In examples of such embodiments, the PIM 104 performs amplification, filtering, and/or aggregating of the data. In an embodiment, the PIM 104 also supplies high- and low-voltage DC power to support operation of the device 102 including circuitry within the scanner assembly 110.

The processing system 106 receives the echo data from the scanner assembly 110 by way of the PIM 104 and processes the data to reconstruct an image of the tissue structures in the medium surrounding the scanner assembly 110. The console 106 outputs image data such that an image of the vessel 120, such as a cross-sectional image of the vessel 120, is displayed on the monitor 108. Vessel 120 may represent fluid filled or surrounded structures, both natural and man-made. The vessel 120 may be within a body of a patient. The vessel 120 may be a blood vessel, as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices.

In some embodiments, the IVUS device includes some features similar to traditional solid-state IVUS catheters, such as the EagleEye^{®} catheter available from Koninklijke Philips N.V. and those disclosed in U.S. Patent No. 7,846,101. For example, the IVUS device 102 includes the scanner assembly 110 near a distal end of the device 102 and a transmission line bundle 112 extending along the longitudinal body of the device 102. The transmission line bundle or cable 112 can include a plurality of conductors, including one, two, three, four, five, six, seven, or more conductors 218 (Fig. 2). Further, in some embodiments, the IVUS device 102 includes a plurality of transmission line bundles each comprising a plurality of conductors of varying size (e.g., gauge), insulation, and/or other structural and electrical characteristics. It is understood that any suitable gauge wire can be used for the conductors 218. In an embodiment, the cable 112 can include a four-conductor transmission line arrangement with, e.g., 0.071 mm diameter (41 AWG gauge) wires. In an embodiment, the cable 112 can include a seven-conductor transmission line arrangement utilizing, e.g., 0.051 mm diameter (44 AWG gauge) wires. In some embodiments, 0.056 mm diameter (43 AWG gauge) wires can be used.

The transmission line bundle 112 terminates in a PIM connector 114 at a proximal end of the device 102. The PIM connector 114 electrically couples the transmission line bundle 112 to the PIM 104 and physically couples the IVUS device 102 to the PIM 104. In an embodiment, the IVUS device 102 further includes a guide wire exit port 116. Accordingly, in some instances the IVUS device is a rapid-exchange catheter. The guide wire exit port 116 allows a guide wire 118 to be inserted towards the distal end in order to direct the device 102 through the vessel 120.

In an embodiment, the processing system 106 generates flow data by processing the echo signals from the IVUS device 102 into Doppler power or velocity information. The processing system 106 may also generate B-mode data by applying envelope detection and logarithmic compression on the conditioned echo signals. The processing system 106 can further generate images in various views, such as 2D and/or 3D views, based on the flow data or the B-mode data. The processing system 106 can also perform various analyses and/or assessments. For example, the processing system 106 can apply virtual histology (VH) techniques, for example, to analyze or assess plaques within a vessel (e.g., the vessel 120). The images can be generated to display a reconstructed color-coded tissue map of plaque composition superimposed on a cross-sectional view of the vessel.

In an embodiment, the processing system 106 can apply a blood flow detection algorithm (e.g., ChromaFlo) to determine the movement of blood flow, for example, by acquiring image data of a target region (e.g., the vessel 120) repeatedly and determining the movement of the blood flow from the image data. The blood flow detection algorithm operates based on the principle that signals measured from vascular tissue are relatively static from acquisition to acquisition, whereas signals measured from blood flow vary at a characteristic rate corresponding to the flow rate. As such, the blood flow detection algorithm may determine movements of blood flow based on variations in signals measured from the target region between repeated acquisitions. To acquire the image data repeatedly, the processing system 106 may control to the device 102 to transmit repeated pulses on the same aperture.

While the present disclosure describes embodiments related to intravascular ultrasound (IVUS) imaging using an intravascular catheter or guidewire, it is understood that one or more aspects of the present disclosure can be implemented in any suitable ultrasound imaging system, including a synthetic aperture ultrasound imaging system, a phased array ultrasound imaging system, or any other array-based ultrasound imaging system. For example, aspects of the present disclosure can be implemented in intraluminal ultrasound imaging systems using an intracardiac (ICE) echocardiography catheter and/or a transesophageal echocardiography (TEE) probe, and/or external ultrasound imaging system using an ultrasound probe configured for imaging while positioned adjacent to and/or in contact with the patient's skin. The ultrasound imaging device can be a transthoracic echocardiography (TTE) imaging device in some embodiments.

An ultrasound transducer array of ultrasound imaging device includes an array of acoustic elements configured to emit ultrasound energy and receive echoes corresponding to the emitted ultrasound energy. In some instances, the array may include any number of ultrasound transducer elements. For example, the array can include between 2 acoustic elements and 10000 acoustic elements, including values such as 2 acoustic elements, 4 acoustic elements, acoustic elements, 64 acoustic elements, 128 acoustic elements, 500 acoustic elements, 812 acoustic elements, 3000 acoustic elements, 9000 acoustic elements, and/or other values both larger and smaller. In some instances, the transducer elements of the array may be arranged in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of transducer elements (e.g., one or more rows, one or more columns, and/or one or more orientations) can be uniformly or independently controlled and activated. The array can be configured to obtain one-dimensional, two-dimensional, and/or three-dimensional images of patient anatomy.

The ultrasound transducer elements may include piezoelectric/piezoresistive elements, piezoelectric micromachined ultrasound transducer (PMUT) elements, capacitive micromachined ultrasound transducer (CMUT) elements, and/or any other suitable type of ultrasound transducer elements. The ultrasound transducer elements of the array are in communication with (e.g., electrically coupled to) electronic circuitry. For example, the electronic circuitry can include one or more transducer control logic dies. The electronic circuitry can include one or more integrated circuits (IC), such as application specific integrated circuits (ASICs). In some embodiments, one or more of the ICs can include a microbeamformer (µBF). In other embodiments, one or more of the ICs includes a multiplexer circuit (MUX).

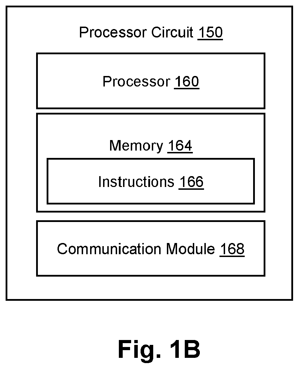

Fig. 1B is a schematic diagram of a processor circuit 150, according to embodiments of the present disclosure. The processor circuit 150 may be implemented in the processing system 106 and/or the imaging device 102 of Fig. 1A. As shown, the processor circuit 150 may include a processor 160, a memory 164, and a communication module 168. These elements may be in direct or indirect communication with each other, for example via one or more buses.

The processor 160 may include a central processing unit (CPU), a digital signal processor (DSP), an ASIC, a controller, an FPGA, another hardware device, a firmware device, or any combination thereof configured to perform the operations described herein. The processor 160 may also be implemented as a combination of computing devices, e.g., a combination of a DSP and a microprocessor, a plurality of microprocessors, one or more microprocessors in conjunction with a DSP core, or any other such configuration.

The memory 164 may include a cache memory (e.g., a cache memory of the processor 160), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 164 includes a non-transitory computer-readable medium. The memory 164 may store instructions 166. The instructions 166 may include instructions that, when executed by the processor 160, cause the processor 160 to perform the operations described herein with reference to the processing system 106 and/or the imaging device 102 (Fig. 1A). Instructions 166 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 168 can include any electronic circuitry and/or logic circuitry to facilitate direct or indirect communication of data between the processor circuit 150, the imaging device 102, and/or the display 108. In that regard, the communication module 168 can be an input/output (I/O) device. In some instances, the communication module 168 facilitates direct or indirect communication between various elements of the processor circuit 150 and/or the processing system 106 (Fig. 1A).

Fig. 2 is a diagrammatic top view of a portion of a flexible assembly 200, according to aspects of the present disclosure. The flexible assembly 200 includes a transducer array 124 formed in a transducer region 204 and transducer control logic dies 206 (including dies 206A and 206B) formed in a control region 208, with a transition region 210 disposed therebetween.

The transducer control logic dies 206 are mounted on a flexible substrate 214 into which the transducers 212 have been previously integrated. The flexible substrate 214 is shown in a flat configuration in Fig. 2. Though six control logic dies 206 are shown in Fig. 2, any number of control logic dies 206 may be used. For example, one, two, three, four, five, six, seven, eight, nine, ten, or more control logic dies 206 may be used.

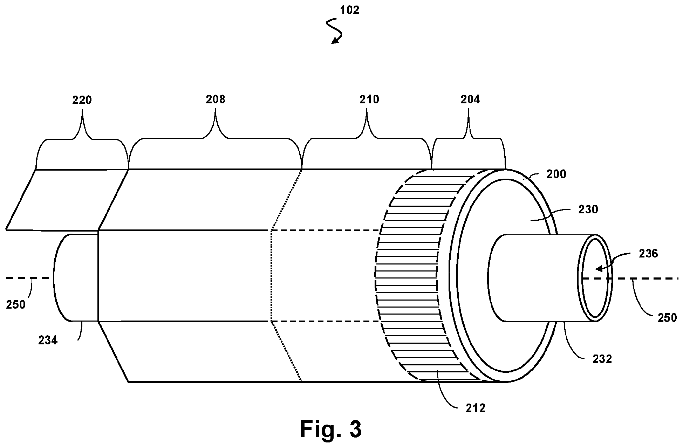

The flexible substrate 214, on which the transducer control logic dies 206 and the transducers 212 are mounted, provides structural support and interconnects for electrical coupling. The flexible substrate 214 may be constructed to include a film layer of a flexible polyimide material such as KAPTON^{™} (trademark of DuPont). Other suitable materials include polyester films, polyimide films, polyethylene napthalate films, or polyetherimide films, liquid crystal polymer, other flexible printed semiconductor substrates as well as products such as Upilex^{®} (registered trademark of Ube Industries) and TEFLON^{®} (registered trademark of E.I. du Pont). In the flat configuration illustrated in Fig. 2, the flexible substrate 214 has a generally rectangular shape. As shown and described herein, the flexible substrate 214 is configured to be wrapped around a support member 230 (Fig. 3) in some instances. Therefore, the thickness of the film layer of the flexible substrate 214 is generally related to the degree of curvature in the final assembled flexible assembly 200. In some embodiments, the film layer is between 5 µm and 100 µm, with some particular embodiments being between 5 µm and 25.1 µm, e.g., 6 µm.

The transducer control logic dies 206 is a non-limiting example of a control circuit. The transducer region 204 is disposed at a distal portion 221 of the flexible substrate 214. The control region 208 is disposed at a proximal portion 222 of the flexible substrate 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or, the length 227 of the transition region 210 may be less than lengths 225 and 229, the length 227 of the transition region 210 can be greater than lengths 225, 229 of the transducer region and controller region, respectively.

The control logic dies 206 are not necessarily homogenous. In some embodiments, a single controller is designated a master control logic die 206A and contains the communication interface for the transmission line bundle 112, which may serve as an electrical communication bus between a processing system, e.g., processing system 106, and the flexible assembly 200. Accordingly, the master control circuit may include control logic that decodes control signals received over the transmission line bundle 112, transmits control responses over the transmission line bundle 112, amplifies echo signals, and/or transmits the echo signals over the transmission line bundle 112. The remaining controllers are slave controllers 206B. The slave controllers 206B may include control logic that drives a transducer 212 to emit an ultrasonic signal and selects a transducer 212 to receive an echo. In the depicted embodiment, the master controller 206A does not directly control any transducers 212. In other embodiments, the master controller 206A drives the same number of transducers 212 as the slave controllers 206B or drives a reduced set of transducers 212 as compared to the slave controllers 206B. In an exemplary embodiment, a single master controller 206A and eight slave controllers 206B are provided with eight transducers assigned to each slave controller 206B.

To electrically interconnect the control logic dies 206 and the transducers 212, in an embodiment, the flexible substrate 214 includes conductive traces 216 formed in the film layer that carry signals between the control logic dies 206 and the transducers 212. In particular, the conductive traces 216 providing communication between the control logic dies 206 and the transducers 212 extend along the flexible substrate 214 within the transition region 210. In some instances, the conductive traces 216 can also facilitate electrical communication between the master controller 206A and the slave controllers 206B. The conductive traces 216 can also provide a set of conductive pads that contact the conductors 218 of the transmission line bundle 112 when the conductors 218 of the transmission line bundle 112 are mechanically and electrically coupled to the flexible substrate 214. Suitable materials for the conductive traces 216 include copper, gold, aluminum, silver, tantalum, nickel, and tin, and may be deposited on the flexible substrate 214 by processes such as sputtering, plating, and etching. In an embodiment, the flexible substrate 214 includes a chromium adhesion layer. The width and thickness of the conductive traces 216 are selected to provide proper conductivity and resilience when the flexible substrate 214 is rolled. In that regard, an exemplary range for the thickness of a conductive trace 216 and/or conductive pad is between 1-5 µm. For example, in an embodiment, 5 µm conductive traces 216 are separated by 5 µm of space. The width of a conductive trace 216 on the flexible substrate may be further determined by the width of the conductor 218 to be coupled to the trace/pad.

The flexible substrate 214 can include a conductor interface 220 in some embodiments. The conductor interface 220 can be a location of the flexible substrate 214 where the conductors 218 of the transmission line bundle 112 are coupled to the flexible substrate 214. For example, the bare conductors of the transmission line bundle 112 are electrically coupled to the flexible substrate 214 at the conductor interface 220. The conductor interface 220 can be tab extending from the main body of flexible substrate 214. In that regard, the main body of the flexible substrate 214 can refer collectively to the transducer region 204, controller region 208, and the transition region 210. In the illustrated embodiment, the conductor interface 220 extends from the proximal portion 222 of the flexible substrate 214. In other embodiments, the conductor interface 220 is positioned at other parts of the flexible substrate 214, such as the distal portion 221, or the flexible substrate 214 may lack the conductor interface 220. A value of a dimension of the tab or conductor interface 220, such as a width 224, can be less than the value of a dimension of the main body of the flexible substrate 214, such as a width 226. In some embodiments, the substrate forming the conductor interface 220 is made of the same material(s) and/or is similarly flexible as the flexible substrate 214. In other embodiments, the conductor interface 220 is made of different materials and/or is comparatively more rigid than the flexible substrate 214. For example, the conductor interface 220 can be made of a plastic, thermoplastic, polymer, hard polymer, etc., including polyoxymethylene (e.g., DELRIN^{®}), polyether ether ketone (PEEK), nylon, Liquid Crystal Polymer (LCP), and/or other suitable materials.

Fig. 3 illustrates a perspective view of the device 102 with the scanner assembly 200 in a rolled configuration. In some instances, the assembly 200 is transitioned from a flat configuration (Fig. 2) to a rolled or more cylindrical configuration (Fig. 3). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND SENSING ASSEMBLY HAVING A FLEXIBLE SUBSTRATE".

In some embodiments, the transducer elements 212 and/or the controllers 206 can be positioned in in an annular configuration, such as a circular configuration or in a polygon configuration, around a longitudinal axis 250 of a support member 230. It will be understood that the longitudinal axis 250 of the support member 230 may also be referred to as the longitudinal axis of the scanner assembly 200, the flexible elongate member 121, and/or the device 102. For example, a cross-sectional profile of the imaging assembly 200 at the transducer elements 212 and/or the controllers 206 can be a circle or a polygon. Any suitable annular polygon shape can be implemented, such as a based on the number of controllers/transducers, flexibility of the controllers/transducers, etc., including a pentagon, hexagon, heptagon, octagon, nonagon, decagon, etc. In some examples, the plurality of transducer controllers 206 may be used for controlling the plurality of ultrasound transducer elements 212 to obtain imaging data associated with the vessel 120.

The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014, ('220 Application). The support member 230 can be a ferrule having a distal flange or portion 232 and a proximal flange or portion 234. The support member 230 can be tubular in shape and define a lumen 236 extending longitudinally therethrough. The lumen 236 can be sized and shaped to receive the guide wire 118. The support member 230 can be manufactured using any suitable process. For example, the support member 230 can be machined and/or electrochemically machined or laser milled, such as by removing material from a blank to shape the support member 230, or molded, such as by an injection molding process.

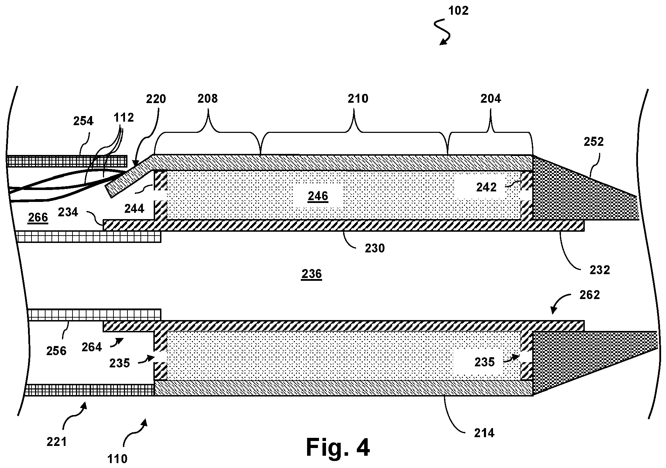

Referring now to Fig. 4, shown there is a diagrammatic cross-sectional side view of a distal portion of the intraluminal imaging device 102, including the flexible substrate 214 and the support member 230, according to aspects of the present disclosure. The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014. The support member 230 can be ferrule having a distal portion 262 and a proximal portion 264. The support member 230 can define a lumen 236 extending along the longitudinal axis LA. The lumen 236 is in communication with the entry/exit port 116 and is sized and shaped to receive the guide wire 118 (Fig. 1A). The support member 230 can be manufactured according to any suitable process. For example, the support member 230 can be machined and/or electrochemically machined or laser milled, such as by removing material from a blank to shape the support member 230, or molded, such as by an injection molding process. In some embodiments, the support member 230 may be integrally formed as a unitary structure, while in other embodiments the support member 230 may be formed of different components, such as a ferrule and stands 242, 244, that are fixedly coupled to one another. In some cases, the support member 230 and/or one or more components thereof may be completely integrated with inner member 256. In some cases, the inner member 256 and the support member 230 may be joined as one, e.g., in the case of a polymer support member.

Stands 242, 244 that extend vertically are provided at the distal and proximal portions 262, 264, respectively, of the support member 230. The stands 242, 244 elevate and support the distal and proximal portions of the flexible substrate 214. In that regard, portions of the flexible substrate 214, such as the transducer portion or region 204, can be spaced from a central body portion of the support member 230 extending between the stands 242, 244. The stands 242, 244 can have the same outer diameter or different outer diameters. For example, the distal stand 242 can have a larger or smaller outer diameter than the proximal stand 244 and can also have special features for rotational alignment as well as control chip placement and connection. To improve acoustic performance, any cavities between the flexible substrate 214 and the surface of the support member 230 are filled with a backing material 246. The liquid backing material 246 can be introduced between the flexible substrate 214 and the support member 230 via passageways 235 in the stands 242, 244. In some embodiments, suction can be applied via the passageways 235 of one of the stands 242, 244, while the liquid backing material 246 is fed between the flexible substrate 214 and the support member 230 via the passageways 235 of the other of the stands 242, 244. The backing material can be cured to allow it to solidify and set. In various embodiments, the support member 230 includes more than two stands 242, 244, only one of the stands 242, 244, or neither of the stands. In that regard the support member 230 can have an increased diameter distal portion 262 and/or increased diameter proximal portion 264 that is sized and shaped to elevate and support the distal and/or proximal portions of the flexible substrate 214.

The support member 230 can be substantially cylindrical in some embodiments. Other shapes of the support member 230 are also contemplated including geometrical, non-geometrical, symmetrical, non-symmetrical, cross-sectional profiles. As the term is used herein, the shape of the support member 230 may reference a cross-sectional profile of the support member 230. Different portions the support member 230 can be variously shaped in other embodiments. For example, the proximal portion 264 can have a larger outer diameter than the outer diameters of the distal portion 262 or a central portion extending between the distal and proximal portions 262, 264. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter.

A proximal inner member 256 and a proximal outer member 254 are coupled to the proximal portion 264 of the support member 230. The proximal inner member 256 and/or the proximal outer member 254 can include a flexible elongate member. The proximal inner member 256 can be received within a proximal flange 234. The proximal outer member 254 abuts and is in contact with the flexible substrate 214. A distal member 252 is coupled to the distal portion 262 of the support member 230. For example, the distal member 252 is positioned around the distal flange 232. The distal member 252 can abut and be in contact with the flexible substrate 214 and the stand 242. The distal member 252 can be the distal-most component of the intraluminal imaging device 102.

One or more adhesives can be disposed between various components at the distal portion of the intraluminal imaging device 102. For example, one or more of the flexible substrate 214, the support member 230, the distal member 252, the proximal inner member 256, and/or the proximal outer member 254 can be coupled to one another via an adhesive.

The conductor interface 220 is positioned at a proximal end of the substrate 214, and provides a point of electrical contact for the transmission line bundle 112. As described above, the transmission line bundle 112 may comprise a plurality of conductors configured to carry signals to and from the electrical components positioned on the substrate. The conductors of the transmission line bundle 112 are sized, shaped, and otherwise configured to be positioned within the space 266 between the proximal outer member 254 and the proximal inner member 256.

As described above, space available within the spaces provided in the elongate body of the catheter (e.g., within the proximal outer member 254) may be limited. One approach to positioning the conductors of the transmission line bundle 112 within the limited spaces of the catheter is to use a single group of small-gauge wires or ribbons spanning an entire length of the catheter from the scanner assembly to the PIM. The conductors of the bundle 112 may be bundled together to form one or more twisted pairs, twisted quads, twisted groups, or other arrangements of conductors. In some embodiments, one or more of the conductors is non-twisted, such that it runs parallel with one or more conductors or twisted groups of conductors. However, the small size of the conductors may result in signal loss and/or restrictions on signal transmission, including negative effects on the bandwidth and/or quality of the electrical signals.

Accordingly, the present disclosure provides multi-segment intraluminal imaging devices that include conductor bundles or segments having different cross-sections sizes positioned within different longitudinal sections of the intraluminal imaging device. In that regard, the intraluminal imaging devices of the present disclosure include elongate components arranged to define inner spaces of different sizes along different sections of the length. Accordingly, larger conductors are included in at least one section of the intraluminal imaging device, where the segment or section defines an inner space of a larger size compared to other segments at other longitudinal positions along the length of the device. By including the larger conductors in the one or more segments of the device that have more available space, the electrical signals carried by the conductors can exhibit less signal loss.

It will be understood that, while the embodiments described below include IVUS imaging catheters, the present disclosure contemplates that the described structural features and/or arrangements may be used in other types of intraluminal devices, including sensing catheters, guide catheters, imaging probes, sensing probes, or any other suitable type of device.

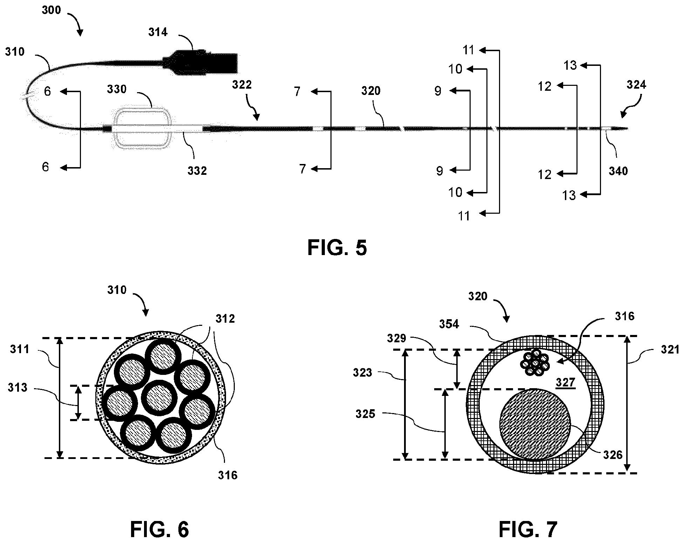

Fig. 5 is a top plan view of an IVUS imaging catheter 300, according to some embodiments of the present disclosure. The catheter 300 includes a cable 310, which is coupled to a proximal connector 314 at the proximal end of the cable 310. The proximal connector 314 may also be referred to as an electrical connector, connector, or PIM connector. The catheter 300 further includes a flexible elongate member 320 configured to be inserted into a body lumen of the patient, such as a blood vessel. An imaging assembly 340 or scanner assembly is coupled to a distal portion 324 of the flexible elongate member 320. A proximal portion of the flexible elongate member 320 is coupled to a hub 330. A distal portion of the cable 310 is also coupled to the hub 330.

The cable 310 may include a flexible sheath, insulation, and/or shielding surrounding a plurality of individual conductors. The conductors may extend within the cable 310 and into the hub 330. In some embodiments, the hub 330 comprises a hollow body or housing defining a space in which the plurality of conductors can be positioned. In some embodiments, the IVUS imaging catheter 300 further includes a hub tubing 332 disposed at least partially within the hub 330 and configured to provide a channel or lumen through which the conductors and/or other components extend, including fluid lines, for example. In some embodiments, the hub 330 forms a handle or handling feature configured to be gripped by a user to control movement of the distal portion 324 of the IVUS imaging catheter 300. Further, in some embodiments, the space within the hub 330 may be used to position respective portions of different conductor bundles. In some embodiments, different segments or bundles of conductors are electrically coupled in the space within the hub 330.

As described further below with respect to Figs. 6, 7, and 9-13, the imaging assembly 340 and the electrical connector 314 are electrically coupled via multiple segments of electrical conductors positioned within different respective sections of the IVUS imaging device, including within the cable 310, and the flexible elongate member 320. In an exemplary embodiment, the cable 310 includes a first segment or bundle of conductors and the flexible elongate member 320 includes at least a second segment or bundle of conductors. The first segment may include conductors having a first cross-sectional size, and the second segment may include conductors having a smaller second cross-sectional size.

Fig. 6 is a diagrammatic cross-sectional view of the cable 310 of the IVUS imaging catheter 300 shown in Fig. 5, taken along line 6-6. The cable 310 includes a flexible sheath or insulation 302, and a first plurality or bundle of conductors 312 forming a first conductor segment is positioned within an interior or lumen of the sheath 302. In that regard, an inner diameter 311 of the sheath 302 defines a space in which the conductors 312 are positioned. In some embodiments, the cable 310 includes additional components that surround one or more of the conductors 312, including electrical shielding, additional insulation, structural support layers, jackets, etc. The components of the cable 310, including the sheath 302 and the conductors 312, may be flexible such that the cable 310 can be maneuvered and easily moved by a physician. In the illustrated embodiment, the cable 310 comprises a substantially circular cross section. However, it will be understood that, in other embodiments, the cable 310 may comprise other cross sections or shapes, including rectangular, polygonal, elliptical, and/or any other suitable cross section or combinations thereof.

In the illustrated embodiment, the cable 310 includes eight conductors 312, wherein each conductor 312 comprises an individual insulation layer. However, the cable 310 may include fewer or more conductors 312, including one, two, three, four, five, six, ten, fifteen, twenty, thirty, or any other suitable number of conductors, both greater and smaller. In some embodiments, the insulation of each conductor 312 comprises a polymer jacket or sheath positioned around a metallic core. In other embodiments, the insulation comprises a coating. The eight conductors 312 form a transmission line bundle that substantially fills the internal space available for the conductors 312. In other embodiments, the conductors 312 fill only a portion of the internal space available within the cable 310.

In some embodiments, two or more of the conductors 312 form twisted pairs, twisted triplets, twisted quads, or any other suitable arrangement. In an exemplary embodiment, the conductors 312 form two twisted quads, wherein each quad includes four conductors 312. In some aspects, the twisted quad may be arranged as a star quad. In some embodiments, the bundle of conductors 312 includes a combination of twisted groups and individual conductors 312. For example, in some embodiments, a power line, or high voltage conductor, may extend individually through the cable 310 alongside a twisted quad and/or a twisted pair.

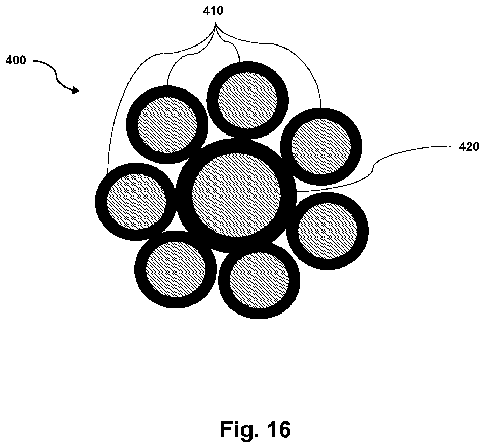

The conductors 312 each comprise a cross-sectional size or area, which may be associated with or based on a diameter 313 of the conductor 312. In some embodiments, the size or diameter 313 of each of the conductors 312 may be 0.127 mm (36 AWG). However, it will be understood that this value is merely exemplary and may vary without departing from the scope of this disclosure. For example, in some embodiments, the diameter 313 may have a value ranging from approximately 0.051 mm (44AWG) to approximately 0.203 mm (32 AWG), including values such as 0.127 mm (36 AWG), 0.079 mm (40 AWG), or any other suitable value, both larger and smaller. In some embodiments, the conductors 312 each comprise a same diameter 313. In other embodiments, one or more of the conductors comprises a diameter that is different from the diameters of one or more of the other conductors 312. For example, in one embodiment (see, e.g., Fig. 16), a power line or high-voltage conductor may comprise a larger diameter than the diameters of the other conductors 312. The conductors 312 may be formed of, or include, one or more metallic materials, including copper, gold, silver, a metal alloy, and/or any other suitable metallic material or combination thereof.

The construction of the cable 310, which unlike the flexible elongate member 320, is not configured to be inserted into the patient's vasculature, does not include the same internal components for mechanical performance as the flexible elongate member 320, and therefore the space provided within the cable 310 for the conductors 312 may be larger than the space provided within the flexible elongate member 320. In some aspects, the increased space within the cable 310 relative to the flexible elongate member 320 is at least partially a result of a larger inner diameter 311. However, in other embodiments, there may be more space for conductors 312 within the cable 310 even if the inner diameter 311 of the cable 310 is smaller than that of the flexible elongate member 320. Accordingly, the conductors 312 positioned within the cable 310 can have a larger cross-sectional size (e.g., diameter) than the bundle of conductors 316 positioned within the flexible elongate member 320.

Fig. 7 is a diagrammatic cross-sectional view of the flexible elongate member 320 of the IVUS device 300 shown in Fig. 5, taken along line 7-7. Although the cross section shown in Fig. 7 is depicted as approximately the same size as the cross section shown in Fig. 6, it will be understood that the diagrammatic views of Figs. 6 and 7 are not necessarily to scale, and that the relative dimensions of the illustrated features may vary from what is depicted. The flexible elongate member 320 includes an outer member 354, which may include, or be referred to as, a catheter, a sheath, tubing, and/or cannula. In the illustrated embodiment, the outer member 354 comprises a circular cross section. However, the outer member 354 may include other shapes or cross-sections, including rectangular, triangular, elliptical, polygonal, multi-luminal and/or any other suitable shape or combination thereof. In the illustrated embodiment, the outer member 354 comprises an outer diameter 321, and an inner diameter 323 at least partially defining a lumen or space 327 in which a second bundle of conductors 316 is disposed. In some embodiments, the outer diameter 321 of the outer member 354 may be 0.89 mm (0.035 in.), and the inner diameter 323 of the outer member 354 may be 0.71 mm (0.028 in.), such that a thickness of the outer member 354 is approximately 0.18 mm (0.007 in.). However, it will be understood that these values are merely exemplary and may vary without departing from the scope of this disclosure. For example, in some embodiments, the outer diameter 321 and/or inner diameter 323 may have a value ranging from approximately 0.01 in. to approximately 0.1 in., including values such as 0.38 mm (0.015 in.), 0.51 mm (0.020 in.), 0.64 mm (0.025 in.), 0.76 mm (0.03 in.), 1.02 mm (0.04 in.), 1.52 mm (0.06 in.), 2.03 mm (0.08 in.), or any other suitable value, both larger and smaller. Further, it will be understood that, in some embodiments, the outer diameter 321 and/or the inner diameter 323 of the outer member 354 may vary across its length. In other embodiments, the outer diameter 321 and/or inner diameter 323 may be constant or substantially constant across its length. Further, it will be understood that, while the size of the outer member 354, conductors 312, conductors 316, core wire 326, and other components are described in terms of their diameters, other dimensions may also be used to describe the sizes of the various components, including cross-sectional area.

As described further below, due to additional components (e.g., core wire 326) within the outer member 354, the conductors 316 disposed within the flexible elongate member 320 have smaller cross-sectional sizes than the conductors 312. The conductors 316 may be sized such that they fit within outer member 354 across its entire length, or a portion of its length, in the space provided between the inner surface of the outer member 354 and other components within the outer member 354. For example, in some embodiments, the diameter of one or more of the conductors 316 may be 0.056 mm (43 AWG). However, it will be understood that this value is merely exemplary and may vary without departing from the scope of this disclosure. For example, in some embodiments, the diameter may have a value ranging from approximately 0.079 mm (40 AWG) to approximately 0.026 mm (50 AWG), including values such as 0. 051 mm (44 AWG), 0.031 mm (48 AWG), or any other suitable value, both larger and smaller.

At the cross section shown in Fig. 7, the lumen or space 327 is also defined by the presence of a core wire 326 positioned within the outer member 354. The core wire 326 may comprise a metallic and/or polymeric wire or member, and may provide structural support, rigidity, and/or other mechanical properties to affect the mechanical performance of the flexible elongate member 320. The core wire 326 of the embodiment shown in Fig. 7 includes a circular cross section. However, the core wire 326 may include other shapes or types of cross-sections, including rectangular, triangular, elliptical, polygonal, and/or any other suitable shape or combination thereof. In some embodiments, the core wire 326 includes an insulation or coating. In other embodiments, the core wire 326 does not include an insulation or coating. In some embodiments, the core wire 326 is formed of a single material. In other embodiments, the core wire 326 is formed of multiple materials. For example, the core wire 326 may include multiple concentric layers of different materials. Materials used by the core wire 326 may be metallic, such as stainless steel, nitinol, or other alloys. In other embodiments, the core wire 326 may include one or more polymeric materials.

The core wire 326 comprises a diameter 325, which may be associated with a cross-sectional area occupied by the core wire 326 within the outer member 354. In some embodiments, the diameter 325 of the core wire 326 may be 0.018 in. at the cross section shown in Fig. 7. However, it will be understood that this value is merely exemplary and may vary without departing from the scope of this disclosure. For example, in some embodiments, the diameter 325 may have a value ranging from approximately 0.03 mm (0.001 in.) to approximately 2.54 mm (0.1 in.), including values such as 0.26 mm (0.01 in.), 0.38 mm (0.015 in.), 0.51 mm (0.02 in.), 0.76 mm (0.03 in.), 1.27 mm (0.05 in.), or any other suitable value, both larger and smaller. In some aspects, the space 327 available for the conductors 316 may be described with respect to a clearance 329, which is the distance or gap between the outer surface of the core wire 326 and the inner surface of the outer member 354. It may be advantageous to increase the clearance 329 at one or more regions or sections along the length of the flexible elongate member 320 to allow for larger segments or bundles of conductors 316. For example, at the cross section shown in Fig. 7, a clearance 329 of approximately 0.26 mm (0.01 in.) is available for conductors 316. The size of the conductors used in this region may be at least partially based on the clearance 329.

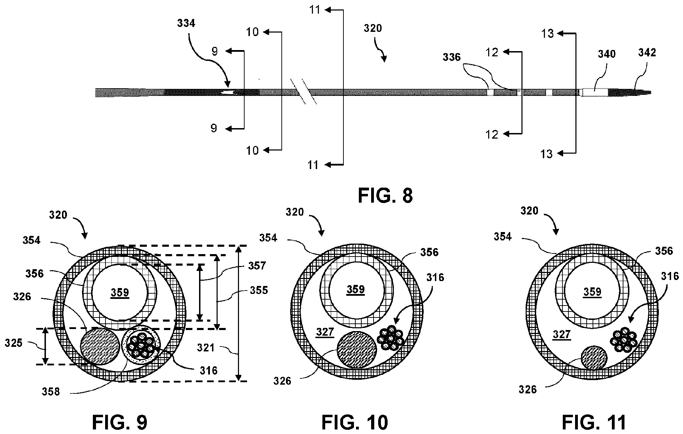

As shown further below with respect to Figs. 9, 10, and 11, the outer diameter 325 of the core wire 326 vary along its length. For example, the core wire 326 may be tapered in one or more longitudinal sections of the core wire 326. In some embodiments, the core wire 326 is tapered and forms a point at a distal end. In some embodiments, one or more intermediate sections of the core wire 326 include a reduced outer diameter 325. The reduced diameter section(s) of the core wire 326 may provide additional space within the lumen 327 for other components, such as a flexible inner member configured to receive a wire, reinforcing components, and/or conductor segments having conductors of larger cross-sectional sizes.

Fig. 8 is a magnified top plan view of a distal region of the IVUS imaging catheter 300 shown in Fig. 5, according to aspects of the present disclosure. In particular, Fig. 8 depicts a guidewire port 334, which may also be referred to as a rapid exchange port, positioned at the distal portion 324 of the flexible elongate member 320, proximal of the scanner assembly 340 and the distal tip member 342. As described below, in some embodiments, the guidewire port 334 is coupled to or in communication with an inner lumen of a flexible inner member (e.g., 356, Fig. 9), such that a guidewire may extend into the guidewire port 334, through the distal portion 324 of the flexible elongate member 320 and the scanner assembly 340, and to the distal tip member 342. Further, the flexible elongate member 320 includes a plurality of radiopaque markers 336 positioned at the distal portion, proximal of the scanner assembly 340. In some embodiments, one or more of the radiopaque markers 336 are positioned around and/or attached to an outer surface of the outer member 354. In some embodiments, one or more of the radiopaque markers 336 are positioned around and/or attached to an outer surface of an inner member (e.g., 356, Fig. 9). Accordingly, in some embodiments, one or more of the radiopaque markers 336 may limit or otherwise affect the space 327 available for the conductors 316.

Fig. 9 is a diagrammatic cross-sectional view of the flexible elongate member 320 of the IVUS device 300 shown in Figs. 5 and 8, taken along line 9-9. The cross section of Fig. 9 is distal of the guidewire port 334. Accordingly, at the cross section of Fig. 9, the flexible elongate member further includes a flexible inner member 356 positioned within the outer member 354. The inner member 356 may be sized, shaped, and otherwise structurally arranged to receive a guidewire. In that regard, the inner member 356 includes an outer diameter 355 and an inner diameter 357, where the inner diameter 357 at least partially defines a guidewire lumen 359. In some embodiments, the outer diameter 355 of the inner member may be 0.02 in. and the inner diameter 357 may be 0.017 in. at the cross section shown in Fig. 7. However, it will be understood that these values are merely exemplary and may vary without departing from the scope of this disclosure. For example, in some embodiments, the outer diameter 355 and/or the inner diameter 357 may have a value ranging from approximately 1.13 mm (0.005 in.) to approximately 2.29 mm (0.09 in.), including values such as 0.26 mm (0.01 in.), 0.38 mm (0.015in.), 0.51 mm (0.02 in.), 0.76 mm (0.03 in.), 1.27 mm (0.05 in.), or any other suitable value, both larger and smaller.

Further, a conductor support member 358 is positioned within the outer member 354, and the bundle of conductors 316 is positioned within the conductor support member 358. In some embodiments, the conductor support member 358 may be configured to house and/or protect the conductors 316 in a region extending across and/or proximate to the guidewire port 334. Thus, the conductors 316 are sized, shaped, and otherwise structurally arranged to fit within the conductor support member 358. In some embodiments, the diameter of one or more of the conductors 316 may be 0.056 mm (43 AWG). However, it will be understood that this value is merely exemplary and may vary without departing from the scope of this disclosure. For example, in some embodiments, the diameter may have a value ranging from approximately 0.079 mm (40 AWG) to approximately 0.026 mm (50 AWG), including values such as 0.051 mm (44 AWG), 0.031 mm (48 AWG), or any other suitable value, both larger and smaller. In some embodiments, the conductors 312 each comprise a same diameter 313. In other embodiments, one or more of the conductors comprises a diameter that is different from the diameters of one or more of the other conductors 312.

In some aspects, the interior of the conductor support member 358 may define a space of smallest cross-sectional area in which the conductors 316 are positioned. Accordingly, in some aspects, the conductors 316 shown in Fig. 9 may be selected such that they can fit, as a bundle, within the interior of the conductor support member, even though the space 327 available to the conductors 316 may be larger at other cross sections, as described below. In some aspects, the conductor support member 358 spatially secures or fixes the conductors 316 relative to other structures at specific locations along the outer member 354. In some aspects, the conductor support member 358 provides additional structural support in certain locations, such as near the guidewire port 334, and may protect the conductors 316 during assembly processes.

The core wire 326 is also shown at the cross section in Fig. 9. In Fig. 9, the outer diameter 325 of the core wire 326 is smaller than the diameter 325 at the cross section of Fig. 7. For example, at the cross section of Fig. 9, the diameter 325 may be approximately 0.26 mm (0.01 in.), in some embodiments. The reduced diameter 325 may be based on a tapering of the core wire 326 across a length of the device. Accordingly, sufficient space is available within the outer member 354 for the inner member 356 in addition to the conductor support member 358, within which the conductors 316 extend.

Fig. 10 is a diagrammatic cross-sectional view of the flexible elongate member 320 of the IVUS device 300 shown in Figs. 5 and 8, taken along line 10-10. At the cross section of Fig. 10, the flexible elongate member 320 includes the outer member 354, the inner member 356, the core wire 326, and the bundle of conductors 316, but does not have the conductor support member 358. In that regard, the conductor support member 358 shown in Fig. 9 does not extend to the location of the cross section of Fig. 10. However, in other embodiments, the conductor support member 358 may extend to the cross section of Fig. 10, or even distal of the cross section of Fig. 10, including to the cross section shown in Fig. 11.

Fig. 11 is a diagrammatic cross-sectional view of the flexible elongate member 320 of the IVUS device 300 shown in Fig. 5, taken along line 11-11. At the cross section of Fig. 11, the flexible elongate member 320 includes the same components as shown in Fig. 10, including the outer member 354, the inner member 356, the core wire 326, and the bundle of conductors 316. In the illustrated embodiment, the outer diameter 325 of the core wire 326 is smaller at the cross section of Fig. 10 relative to the cross section of Fig. 9. Further, the outer diameter 325 of the core wire 326 is smaller at the cross section of Fig. 11 relative to the cross section of Fig. 10. Further, with the conductor support member 358 absent from this section of the flexible elongate member, additional space or clearance in the lumen 327 may be available for the conductors. Accordingly, in some embodiments, a different third bundle or segment of conductors is advantageously positioned within one or more of the sections of the flexible elongate member 320 shown in Figs. 10 and 11 to take advantage of the increased space within the lumen 327. Accordingly, the conductors of the third segment may comprise cross-sectional areas that are larger than the conductors of the second segment.

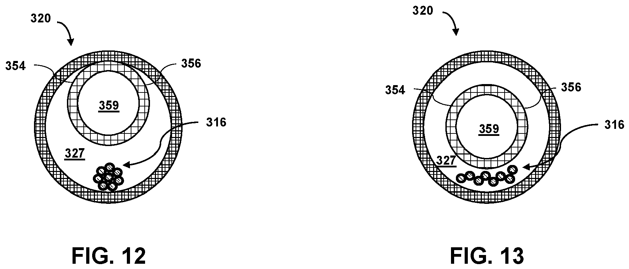

Fig. 12 is a diagrammatic cross-sectional view of the flexible elongate member 320 of the IVUS device 300 shown in Figs. 5 and 8, taken along line 12-12. At the cross section of Fig. 12, the flexible elongate member 320 includes the outer member 354, the inner member 356, and the bundle of conductors 316, but does not have the core wire 326. In that regard, the core wire 326 does not extend to the location of the cross section of Fig. 12. For example, in some embodiments, the core wire 326 tapers to a point, and ends at a location proximal to the cross section of Fig. 12. However, in other embodiments, the core wire 326 may extend to the cross section of Fig. 12, or even distal of the cross section of Fig. 12, including to the cross section shown in Fig. 13. With the core wire 326 absent from the flexible elongate member at the cross section of Fig. 12, additional space is provided for the conductors 316. Accordingly, in some embodiments, a different bundle or segment of larger conductors is advantageously positioned within the section of the flexible elongate member 320 shown in Fig. 12 to take advantage of the increased space within the lumen 327. In some embodiments, the bundle of conductors at the cross section of Fig. 12 is the same bundle or segment of conductors positioned at the cross sections of Figs. 10 and/or 11. In some embodiments, the bundle or segment of conductors at the cross section of Fig. 12 is the same bundle or segment of conductors that extends to the cross section of Fig. 9. In some embodiments, the bundle or segment of conductors at the cross section of Fig. 12 is the same bundle or segment of conductors that extends to the cross section of Fig. 7 and/or to the hub 330 shown in Fig. 5.

In some embodiments a first bundle or segment of conductors having a first cross-sectional area is positioned to extend through the cable 310 to the hub 330, and a different, second bundle of conductors having a smaller, second cross-sectional area is positioned to extend distally of the hub 330. Further, in some embodiments, the second bundle of conductors having the smaller, second cross-sectional area is positioned to extend between the hub 330 and the guidewire port 334, and a different, third bundle of conductors having a third cross-sectional area that may be larger or smaller than the conductors of the second bundle is positioned at the distal portion 324 of the flexible elongate member, distally of the guidewire port 334. As will be explained further below, the conductors of the different bundles or segments may be electrically coupled in any suitable way, including soldering, crimping, mechanical connectors, welding, conductive epoxy, etc.

Fig. 13 is a cross-sectional view of the flexible elongate member 320 of the IVUS device 300 shown in Figs. 5 and 8, taken at line 1313. In that regard, the cross section of Fig. 13 is proximate to the scanner assembly 340 at the distal portion of the flexible elongate member 320. Accordingly, the inner member 356 may be concentric with, or substantially concentric with, the outer member 354, since it is near the connection point with the support member of the scanner assembly (see, e.g., Fig. 4, support member 230, proximal outer member 254, proximal inner member 256). In some aspects, the conductors of the bundle 316 may be arranged to be electrically coupled to a conductor interface of a circuit assembly coupled to a substrate. It will be understood that the clearance of the space 327 may be reduced in the cross section of Fig. 13 relative to the cross section of Fig. 12.

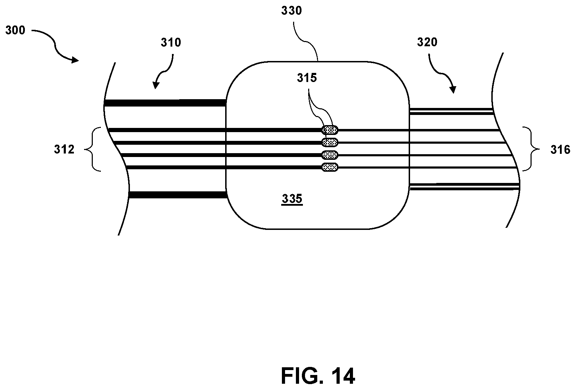

Fig. 14 is a diagrammatic view of a hub 330 of an IVUS catheter 300 forming an electrical connection point for different bundles of conductors having different sizes, according to one embodiment. In that regard, the hub 330 may comprise a housing defining an internal chamber or space 335 configured to receive a plurality of conductors. The hub 330 is coupled to, and disposed between, a cable 310 and a flexible elongate member 320. A first plurality or bundle 312 of conductors extending within the cable 310 enters the hub 330 at a proximal end of the hub 330. A second plurality or bundle 316 of conductors extending within the flexible elongate member 320 enters the hub 330 at a distal end of the hub 330. The conductors of the first plurality or bundle 312 comprise a first size, gauge, cross section, and/or diameter suitable for the cable 310, and the conductors of the second plurality or bundle comprise a second size, gauge, cross section, and/or diameter suitable to be positioned within one or more sections of the flexible elongate member 320. In that regard, in the embodiment of Fig. 14, the conductors of the first plurality 312 are larger, wider, and/or thicker than the conductors of the second plurality 316. The second plurality of conductors 316 may be smaller than the conductors of the first plurality 312 to fit in the space provided within the flexible elongate member 320, as described above. In the illustrated embodiment, each conductor of the first plurality of conductors 312 is electrically coupled to a corresponding or respective conductor of the second plurality of conductors 316. The conductors 312, 316 may be coupled at connection points 315 using a solder connection, a crimp, thermal bonding, conductive epoxy, heat shrink tubing, mechanical connectors, and/or any other suitable form of electrical coupling. Although four conductors are shown in each plurality, it will be understood that any suitable number of conductors may be used, including one, two, three, five, seven, ten, fifteen, twenty, or any other suitable number, both larger and smaller.

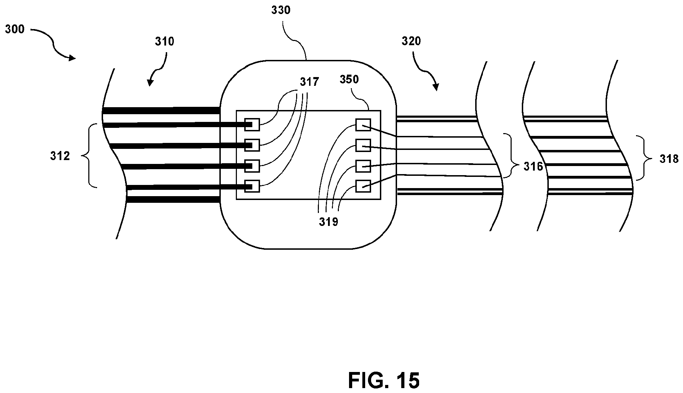

Fig. 15 is a diagrammatic view of a hub 330 of an IVUS catheter 300 forming an electrical connection point for different bundles of conductors having different sizes, according to another embodiment. Similar to the embodiment described with respect to Fig. 14, the IVUS catheter 300 shown in Fig. 15 includes a hub 330, a cable 310, a flexible elongate member 320, a first conductor segment comprising a first plurality of conductors 312 each having a first size, and a second conductor segment comprising a second plurality of conductors 316 each having a second size. The embodiment of Fig. 15 further includes a circuit board 350, which may comprise a printed circuit board (PCB), disposed within and coupled to the hub 330. The circuit board 350 includes a first plurality of electrical contacts 317 corresponding to the first plurality of conductors 312 and a second plurality of electrical contacts 319 corresponding to the second plurality of conductors 316. In the embodiment shown in Fig. 15, each conductor of the first plurality of conductors 312 is coupled to a corresponding conductor of the second plurality of conductors 316 by attaching each conductor of the first plurality 312 to an electrical contact of the first plurality of electrical contacts 317, and attaching each corresponding conductor of the second plurality of conductors 316 to a corresponding electrical contact of the second plurality of electrical contacts 319. In some embodiments, the conductors are electrically coupled to the electrical contacts 317, 319 by soldering, electrical epoxy, electrical terminals, plugs, or any other suitable method of electrical coupling.

Further, in the embodiment of Fig. 15, a third conductor segment comprising a third plurality of conductors 318 is positioned within the flexible elongate member 320 at a distal region of the flexible elongate member 320. The conductors of the third plurality of conductors 318 each comprise a third cross-sectional size, which is larger than the second cross-sectional size of the conductors of the second plurality 316. In that regard, the third plurality or bundle of conductors 318 is sized to fit within a corresponding space provided within the flexible elongate member at the distal region. For example, as described above, the third section may extend distally from a guidewire port toward a distal end of the device. In other embodiments, the third section extends along a shorter length proximate to the scanner assembly. In some embodiments, the device may further include a fourth plurality of conductors of a fourth size, a fifth plurality of conductors of a fifth size, and/or a sixth plurality of conductors of a sixth size, positioned within the flexible elongate member at respective sections, wherein the sizes of the individual conductors are selected to be as large as practicable to fit within the flexible elongate member. By taking advantage of the different spaces available within the flexible elongate member 320 at different longitudinal sections, the signal loss of the electrical signals carried by the different conductor segments can be reduced.

In some embodiments, the conductors of the different conductor segments, such as the first conductor segment and the second conductor segment, are impedance balances or matched to reduce undesirable echoes, noise, or other signal artifacts that can result from impedance mismatched conductors.

Fig. 16 is a diagrammatic cross-sectional view of a bundle 400 of conductors, according to some aspects of the present disclosure. In that regard, the bundle 400 of conductors includes a first group 410 of conductors having a first cross-sectional size, and an individual conductor 420 having a larger second cross-sectional size. It will be understood that the features and arrangements described with respect to Fig. 16 may be used in the bundle of conductors 312 within the cable 310, or one or more of the bundles of conductors 316 positioned within the flexible elongate member 320. Accordingly, in the embodiments provided in this disclosure, one or more of the conductors of a bundle or group of conductors may be larger or smaller than one or more other conductors of the bundle. For example, in some embodiments, a bundle of conductors may comprise a high voltage conductor configured to provide high voltage power to one or more electrical components of a scanner assembly, such as an amplifier, a pulser, and/or a transducer element.

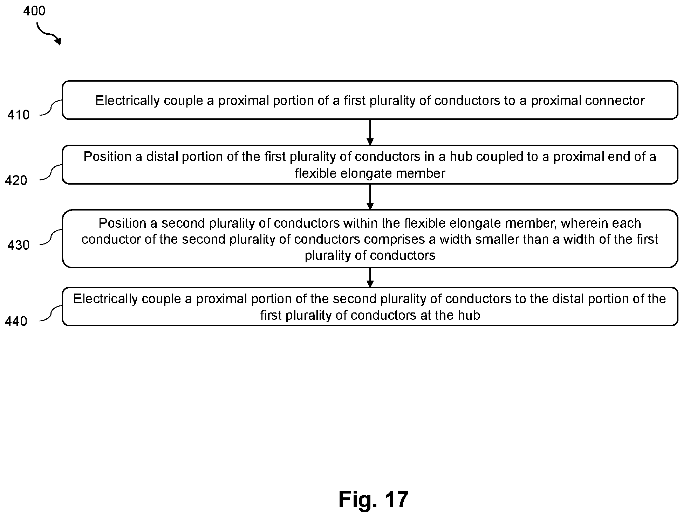

Fig. 17 is a flow diagram illustrating a method 500 for assembling a multi-segment IVUS catheter assembly, according to some embodiments of the present disclosure. In step 510, a proximal portion of a first plurality of conductors is electrically coupled to a proximal connector. In step 520, a distal portion of the first plurality of conductors is positioned within a hub coupled to a proximal end of a flexible elongate member. In step 530, a second plurality of conductors are positioned within the flexible elongate member, wherein each conductor of the second plurality of conductors comprises a width smaller than a width of the first plurality of conductors. In step 540, a proximal portion of the second plurality of conductors is electrically coupled to the distal portion of the first plurality of conductors at the hub.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, illustrative embodiments have been shown and described.

## Claims

1. An intraluminal ultrasound imaging device (300), comprising:
a cable (310) comprising a proximal portion and a distal portion;
an electrical connector (314) coupled to the proximal portion of the cable;
a flexible elongate member (320) comprising a proximal portion and a distal portion, wherein the distal portion of the flexible elongate member is configured to be positioned within a body lumen of a patient, wherein the proximal portion of the flexible elongate member is coupled to the cable;
an ultrasound transducer array (340) coupled to the distal portion of the flexible elongate member;
a first plurality of conductors (312) extending within the cable;
**characterized in that**
a second plurality of conductors (316) is extending within the flexible elongate member,
wherein each conductor of the first plurality of conductors is electrically coupled to a respective conductor of the second plurality of conductors,
wherein a conductor of the first plurality of conductors with a first cross section is electrically coupled to a conductor of the second plurality of conductors with a second cross section that is smaller than the first cross section, and
wherein the first plurality of conductors and the second plurality of conductors are configured to transmit electrical signals between the ultrasound transducer array and the electrical connector.

2. The intraluminal ultrasound imaging device of claim 1, wherein:
the first plurality of conductors comprises a first twisted group of conductors and a first power line, and
the second plurality of conductors comprises a second twisted group of conductors electrically coupled to the first twisted group of conductors, and a second power line electrically coupled to the first power line.

3. The intraluminal ultrasound imaging device of claim 1, wherein the second plurality of conductors comprises eight conductors forming a first twisted quad and a second twisted quad.

4. The intraluminal ultrasound imaging device of claim 1, wherein the second plurality of conductors comprises at least one twisted group of wires, and at least one non-twisted wire.

5. The intraluminal ultrasound imaging device of claim 1, further comprising a hub (330) coupled to the distal portion of the cable and the proximal portion of the flexible elongate member, wherein the first plurality of conductors is electrically coupled to the second plurality of conductors within the hub.

6. The intraluminal ultrasound imaging device of claim 5, wherein the hub comprises:
a housing; and
a printed circuit board positioned within the housing, wherein the printed circuit board comprises a first set of contacts and a second set of contacts, wherein the first plurality of conductors are coupled to the first set of contacts and the second plurality of conductors are coupled to the second set of contacts.

7. The intraluminal ultrasound imaging device of claim 1, wherein the first plurality of conductors and the second plurality of conductors are impedance balanced.

8. The intraluminal ultrasound imaging device of claim 1, further comprising a core wire (326) positioned within the flexible elongate member and defining a space between an outer surface of the core wire and an inner surface of the flexible elongate member, wherein the second plurality of conductors is positioned within the space.

9. The intraluminal ultrasound imaging device of claim 8, further comprising:
a guidewire exit port (116) disposed in the flexible elongate member proximal to the ultrasound transducer array; and
a third plurality of conductors (318) extending distally from the guidewire exit port within the distal portion of the flexible elongate member, wherein a conductor of the third plurality of conductors with a third cross section is electrically coupled to the conductor of the second plurality of conductors with the second cross section, wherein the third cross section is larger than the second cross section.

10. The intraluminal ultrasound imaging device of claim 9, further comprising a flexible inner member (356) positioned within the space and adjacent to the core wire, the flexible inner member extending distally from the guidewire exit port.

11. An intravascular ultrasound, IVUS, imaging system (100), comprising:
an intraluminal ultrasound imaging device according to any of the preceding claims, and
a processor circuit configured to receive and process the electrical signals.

12. The IVUS imaging system of claim 11, wherein the processor circuit is configured to generate an ultrasound image based on the processed electrical signals.

13. The IVUS imaging system of claim 12, wherein the processor circuit is communicatively coupled to display (108), and wherein the processor circuit is configured to output an intraluminal cross-sectional image based on the electrical signals to a display.

14. The IVUS imaging system of claim 11, wherein the processor circuit is configured to perform an analog-to-digital conversion on the electrical signals.

15. The IVUS imaging system of claim 11, further comprising:
a patient interface module (104), PIM, configured to be coupled to the electrical connector of the intraluminal ultrasound imaging device to receive the electrical signals; and
a console communicatively coupled to the PIM and configured to receive the electrical signals from the PIM.

## Patentansprüche

1. Intraluminales Ultraschall-Bildgebungsgerät (300), umfassend: ein Kabel (310), das einen proximalen Abschnitt und einen distalen Abschnitt umfasst; einen elektrischen Verbinder (314), der mit dem proximalen Abschnitt des Kabels verbunden ist; ein flexibles längliches Element (320), das einen proximalen Abschnitt und einen distalen Abschnitt umfasst, wobei der distale Abschnitt des flexiblen länglichen Elements so konfiguriert ist, dass er innerhalb eines Körperlumens eines Patienten positioniert werden kann, wobei der proximale Abschnitt des flexiblen länglichen Elements daran gekoppelt ist das Kabel; eine Ultraschallwandleranordnung (340), die mit dem distalen Abschnitt des flexiblen länglichen Elements gekoppelt ist; eine erste Vielzahl von Leitern (312), die sich innerhalb des Kabels erstrecken; **dadurch gekennzeichnet, dass** eine zweite Mehrzahl von Leitern (316) erstreckt sich innerhalb des flexiblen länglichen Elements, wobei jeder Leiter der ersten Mehrzahl von Leitern elektrisch mit einem jeweiligen Leiter der zweiten Mehrzahl von Leitern gekoppelt ist, wobei ein Leiter der ersten Mehrzahl von Leitern mit einem ersten Querschnitt elektrisch mit einem Leiter der zweiten Mehrzahl von Leitern mit einem zweiten Querschnitt gekoppelt ist, der kleiner als der erste Querschnitt ist, und wobei die erste Mehrzahl von Leitern und die zweite Mehrzahl von Leitern dazu konfiguriert sind, elektrische Signale zwischen der Ultraschallwandleranordnung und dem elektrischen Anschluss zu übertragen.

2. Intraluminales Ultraschall-Bildgebungsgerät nach Anspruch 1, wobei: die erste Mehrzahl von Leitern umfasst eine erste verdrillte Gruppe von Leitern und eine erste Stromleitung, und die zweite Mehrzahl von Leitern umfasst eine zweite verdrillte Gruppe von Leitern, die elektrisch mit der ersten verdrillten Gruppe von Leitern verbunden ist, und eine zweite Stromleitung, die elektrisch mit der ersten Stromleitung verbunden ist.

3. Intraluminales Ultraschallbildgebungsgerät nach Anspruch 1, wobei die zweite Mehrzahl von Leitern acht Leiter umfasst, die ein erstes verdrilltes Viereck und ein zweites verdrilltes Viereck bilden.

4. Intraluminales Ultraschall-Bildgebungsgerät nach Anspruch 1, wobei die zweite Mehrzahl von Leitern mindestens eine verdrillte Gruppe von Drähten und mindestens einen nicht verdrillten Draht umfasst.

5. Intraluminales Ultraschall-Bildgebungsgerät nach Anspruch 1, das außerdem eine Nabe (330) umfasst, die mit dem distalen Abschnitt des Kabels und dem proximalen Abschnitt des flexiblen länglichen Elements gekoppelt ist, wobei die erste Mehrzahl von Leitern elektrisch mit der zweiten Mehrzahl von Leitern darin gekoppelt ist der Hub.

6. Intraluminales Ultraschall-Bildgebungsgerät nach Anspruch 5, wobei die Nabe umfasst: ein Gehäuse; und eine innerhalb des Gehäuses positionierte Leiterplatte, wobei die Leiterplatte einen ersten Satz Kontakte und einen zweiten Satz Kontakte umfasst, wobei die erste Mehrzahl von Leitern mit dem ersten Satz Kontakte und die zweite Mehrzahl von Leitern mit diesem gekoppelt sind der zweite Kontaktsatz.

7. Intraluminales Ultraschall-Bildgebungsgerät nach Anspruch 1, wobei die erste Mehrzahl von Leitern und die zweite Mehrzahl von Leitern impedanzausgeglichen sind.

8. Intraluminales Ultraschall-Bildgebungsgerät nach Anspruch 1, das außerdem einen Kerndraht (326) umfasst, der innerhalb des flexiblen länglichen Elements positioniert ist und einen Raum zwischen einer Außenfläche des Kerndrahts und einer Innenfläche des flexiblen länglichen Elements definiert, wobei die zweite Mehrzahl von Der Leiter wird im Raum positioniert.

9. Intraluminales Ultraschall-Bildgebungsgerät nach Anspruch 8, weiterhin umfassend: eine Führungsdraht-Austrittsöffnung (116), die in dem flexiblen länglichen Element proximal zur Ultraschallwandleranordnung angeordnet ist; und eine dritte Mehrzahl von Leitern (318), die sich distal von der Führungsdraht-Austrittsöffnung innerhalb des distalen Abschnitts des flexiblen länglichen Elements erstrecken, wobei ein Leiter der dritten Mehrzahl von Leitern mit einem dritten Querschnitt elektrisch mit dem Leiter der zweiten Mehrzahl von Leitern gekoppelt ist Leiter mit dem zweiten Querschnitt, wobei der dritte Querschnitt größer ist als der zweite Querschnitt.

10. Intraluminales Ultraschall-Bildgebungsgerät nach Anspruch 9, das außerdem ein flexibles Innenelement (356) umfasst, das innerhalb des Raums und angrenzend an den Kerndraht positioniert ist, wobei sich das flexible Innenelement distal von der Führungsdraht-Austrittsöffnung erstreckt.

11. Intravaskuläres Ultraschall, IVUS Bildgebungssystem (100), umfassend: ein intraluminales Ultraschall-Bildgebungsgerät nach einem der vorhergehenden Ansprüche, und eine Prozessorschaltung, die zum Empfangen und Verarbeiten der elektrischen Signale konfiguriert ist.

12. IVUS-Bildgebungssystem nach Anspruch 11, wobei die Prozessorschaltung so konfiguriert ist, dass sie auf der Grundlage der verarbeiteten elektrischen Signale ein Ultraschallbild erzeugt.

13. IVUS-Bildgebungssystem nach Anspruch 12, wobei die Prozessorschaltung kommunikativ mit der Anzeige (108) gekoppelt ist und wobei die Prozessorschaltung so konfiguriert ist, dass sie ein intraluminales Querschnittsbild basierend auf den elektrischen Signalen an eine Anzeige ausgibt.

14. IVUS-Bildgebungssystem nach Anspruch 11, wobei die Prozessorschaltung so konfiguriert ist, dass sie eine Analog-Digital-Umwandlung der elektrischen Signale durchführt.

15. IVUS-Bildgebungssystem nach Anspruch 11, weiterhin umfassend: ein Patientenschnittstellenmodul (104), PIM, das so konfiguriert ist, dass es mit dem elektrischen Anschluss des intraluminalen Ultraschallbildgebungsgeräts gekoppelt werden kann, um die elektrischen Signale zu empfangen; und eine Konsole, die kommunikativ mit dem PIM verbunden und so konfiguriert ist, dass sie die elektrischen Signale vom PIM empfängt.

## Revendications

1. Dispositif d'imagerie ultrasonore intraluminale (300), comprenant: un câble (310) comprenant une partie proximale et une partie distale; un connecteur électrique (314) couplé à la partie proximale du câble; un élément allongé flexible (320) comprenant une partie proximale et une partie distale, la partie distale de l'élément allongé flexible étant configurée pour être positionnée dans une lumière corporelle d'un patient, la partie proximale de l'élément allongé flexible étant couplée au câble; un réseau de transducteurs à ultrasons (340) couplé à la partie distale de l'élément allongé flexible; une première pluralité de conducteurs (312) s'étendant à l'intérieur du câble; **caractérisé en ce que** une deuxième pluralité de conducteurs (316) s'étend à l'intérieur de l'élément allongé flexible, dans lequel chaque conducteur de la première pluralité de conducteurs est couplé électriquement à un conducteur respectif de la deuxième pluralité de conducteurs, dans lequel un conducteur de la première pluralité de conducteurs ayant une première section transversale est couplé électriquement à un conducteur de la seconde pluralité de conducteurs ayant une seconde section transversale qui est plus petite que la première section transversale, et la première pluralité de conducteurs et la seconde pluralité de conducteurs étant configurées pour transmettre des signaux électriques entre le réseau de transducteurs à ultrasons et le connecteur électrique.

2. Dispositif d'imagerie ultrasonore intraluminale selon la revendication 1, dans lequel: la première pluralité de conducteurs comprend un premier groupe torsadé de conducteurs et une première ligne électrique, et la seconde pluralité de conducteurs comprend un second groupe torsadé de conducteurs couplé électriquement au premier groupe torsadé de conducteurs, et une seconde ligne électrique couplée électriquement à la première ligne électrique.

3. Dispositif d'imagerie ultrasonore intraluminale selon la revendication 1, dans lequel la deuxième pluralité de conducteurs comprend huit conducteurs formant un premier quadrilatère torsadé et un deuxième quadrilatère torsadé.

4. Dispositif d'imagerie ultrasonore intraluminale selon la revendication 1, dans lequel la deuxième pluralité de conducteurs comprend au moins un groupe de fils torsadés et au moins un fil non torsadé.

5. Dispositif d'imagerie ultrasonore intraluminale de la revendication 1, comprenant en outre un moyeu (330) couplé à la partie distale du câble et à la partie proximale de l'élément allongé flexible, dans lequel la première pluralité de conducteurs est couplée électriquement à la seconde pluralité de conducteurs à l'intérieur du moyeu.

6. Dispositif d'imagerie ultrasonore intraluminale selon la revendication 5, dans lequel le moyeu comprend: un boîtier; et une carte de circuit imprimé positionnée à l'intérieur du boîtier, la carte de circuit imprimé comprenant un premier ensemble de contacts et un deuxième ensemble de contacts, la première pluralité de conducteurs étant couplée au premier ensemble de contacts et la deuxième pluralité de conducteurs étant couplée au deuxième ensemble de contacts.

7. Dispositif d'imagerie ultrasonore intraluminale selon la revendication 1, dans lequel la première pluralité de conducteurs et la seconde pluralité de conducteurs sont à impédance équilibrée.

8. Dispositif d'imagerie ultrasonore intraluminale selon la revendication 1, comprenant en outre un fil central (326) positionné à l'intérieur de l'élément allongé flexible et définissant un espace entre une surface externe du fil central et une surface interne de l'élément allongé flexible, dans lequel le second une pluralité de conducteurs est positionnée dans l'espace.

9. Dispositif d'imagerie ultrasonore intraluminale selon la revendication 8, comprenant en outre: un orifice de sortie de fil guide (116) disposé dans l'élément allongé flexible à proximité du réseau de transducteurs à ultrasons; et une troisième pluralité de conducteurs (318) s'étendant de manière distale depuis l'orifice de sortie du fil-guide à l'intérieur de la partie distale de l'élément allongé flexible, dans lequel un conducteur de la troisième pluralité de conducteurs ayant une troisième section transversale est électriquement couplé au conducteur de la deuxième pluralité de conducteurs des conducteurs ayant la deuxième section, la troisième section étant plus grande que la deuxième section.

10. Dispositif d'imagerie ultrasonore intraluminale selon la revendication 9, comprenant en outre un élément interne flexible (356) positionné dans l'espace et adjacent au fil central, l'élément interne flexible s'étendant de manière distale depuis l'orifice de sortie du fil guide.

11. Système d'imagerie à ultrasons intravasculaire, IVUS (100), comprenant: un dispositif d'imagerie ultrasonore intraluminale selon l'une quelconque des revendications précédentes, et un circuit processeur configuré pour recevoir et traiter les signaux électriques.

12. Système d'imagerie IVUS selon la revendication 11, dans lequel le circuit processeur est configuré pour générer une image ultrasonore sur la base des signaux électriques traités.

13. Système d'imagerie IVUS selon la revendication 12, dans lequel le circuit processeur est couplé en communication à un affichage (108), et dans lequel le circuit processeur est configuré pour émettre une image en coupe intraluminale basée sur les signaux électriques vers un affichage.

14. Système d'imagerie IVUS selon la revendication 11, dans lequel le circuit processeur est configuré pour effectuer une conversion analogique-numérique sur les signaux électriques.

15. Système d'imagerie IVUS selon la revendication 11, comprenant en outre: un module d'interface patient (104), PIM, configuré pour être couplé au connecteur électrique du dispositif d'imagerie ultrasonore intraluminale pour recevoir les signaux électriques; et une console couplée en communication au PIM et configurée pour recevoir les signaux électriques du PIM.
